# EUROPEAN PATENT APPLICATION

(11) **EP 4 008 767 A1**
(43) Date of publication of application: **08.06.2022**
(21) Application number: 20849557.2
(22) Date of filing: 29.07.2020
(51) Int. Cl.: C12C 5/02, A23L 27/21, A23L 27/24, A23L 2/00, A23L 2/52, C12G 3/04

(54) **COMPOSITION FOR FOODS AND BEVERAGES AND METHOD FOR PRODUCING SAID COMPOSITION**

(30) Priority: 02.08.2019 JP 2019143376
(71) Applicant: Suntory Holdings Limited, Osaka 530-8203 (JP)
(72) Inventor: SAWADA, Miho, Shimamoto-gun, Osaka (JP); TAKII, Shinya, Kyoto-shi, Kyoto (JP)
(74) Representative: Vossius & Partner Patentanwälte Rechtsanwälte mbB
(86) International application number: PCT/JP2020/029016
(87) International publication number: WO 2021/024874

(57) **Abstract**

A composition for foodstuff, having a mass ratio of a total content of D-isoleucine, D-leucine, and D-valine to the content of D-alanine (D-isoleucine, D-leucine, and D-valine / D-alanine) of 0.1 or more. The composition of the present invention is useful in the fields of foodstuff, and the like.

## Description

### TECHNICAL FIELD

The present invention relates to a composition for foodstuff and a method for producing the same.

### BACKGROUND ART

With the diversification of the inclinations of the consumers in the recent years, the development of beer-taste beverages having various flavor characteristics has been desired. For example, Patent Publication 1 discloses the use of a fermentation rice extract for providing a non-fermented beer-taste nonalcoholic beverage having excellent full-bodied taste and sharp taste, and inclinations, and having a low purine content.

In addition, various seasonings have been proposed for the purposes of improving the tastes of the foodstuff. For example, Patent Publication 2 discloses the use of a mixture of D-alanine, D-aspartic acid, D-glutamic acid, and D-proline, for improving richness of the foodstuff, improving sustainability, and improving taste of stewing (slow cooking).

### RELATED ART REFERENCES

### PATENT PUBLICATIONS

Patent Publication 1: Japanese Patent Gazette No. 6042490
Patent Publication 2: Japanese Patent Gazette No. 6449418

### SUMMARY OF THE INVENTION

### PROBLEMS TO BE SOLVED BY THE INVENTION

However, it could be seen that when the fermentation rice extract of Patent Publication 1 or the mixture of D-amino acids of Patent Publication 2 is used in a beer-taste beverage, the sharpness may be undesirably worsened.

The present invention relates to the provision of a composition for foodstuff capable of giving sharpness to a beer-taste beverage and a method for producing the same.

### MEANS TO SOLVE THE PROBLEMS

The present invention relates to the followings [1] to [2]:
[1] A composition for foodstuff, having a mass ratio of a total content of D-isoleucine, D-leucine, and D-valine to the content of D-alanine (D-isoleucine, D-leucine, and D-valine / D-alanine) of 0.1 or more.
[2] A method for producing a composition for foodstuff as defined in [1], including fermenting a malt extract with *Lb. fermentum.*

### EFFECTS OF THE INVENTION

According to the present invention, a composition for foodstuff capable of giving sharpness to a beer-taste beverage and a method for producing the same can be provided.

### MODES FOR CARRYING OUT THE INVENTION

In general, in the beer-taste beverages, when the sharpness is high, the inclination degree of the customers is improved. The present inventors have newly found that specified D-amino acids such as D-isoleucine, D-leucine, and D-valine contribute to the sharpness of beer-taste beverages. In addition, the inventors have newly found that when the content of D-alanine in the beer-taste beverage becomes large, the sharpness is likely to be worsened. The term "sharpness" as used herein refers to a refreshing taste and a stimulating taste with a light taste at the back of the throat with little lingering aftertaste of sweetness or sweet aroma. The term "beer-taste beverage" as used herein refers to a carbonated beverage having a beer-like flavor. In other words, the beer-taste beverage of the present specification embraces all the carbonated beverages having a beer flavor, unless specified otherwise. The composition for foodstuff of the present invention can be used in beer-taste beverages in general, which can be suitably used also in beverages with light tastes, such as beer-taste alcoholic beverages having a low malt ratio, or low malt-derived ingredient, raw wort extract, real extract, purine content, or alcoholicity, and nonalcoholic beer-taste beverages similarly having a low malt ratio, or low malt-derived ingredient, raw wort extract, real extract, or purine content. Among them, the nonalcoholic beer-taste beverage embraces any of the nonalcoholic carbonated beverages having a beer flavor, irrespective of the presence or absence of a fermentation step with an yeast, unless specified otherwise. In addition, the term "malt ratio" refers to a ratio of the mass of malts occupied in the raw materials other than water and hops, such as malts, rice, maize, great millet, potatoes, starch, and wheat other than malts, and sugars. Here, the ingredient which can be added in a very small amount such as an acidulant, a sweetener, a bittering agent, a seasoning, or a flavor is not included in the calculation of the above ratio. The malt ratio as used herein means a value as calculated in accordance with the Notice of Interpretations of Liquor Taxation Laws and Liquor Governmental Ordinance or the like enforced on April 1, 2018. In general, the raw malt extract has the technical idea of expressing the richness of beers calculated from two parameters: alcohols and extracts (Ingredients of Brewed Products, page 184, edited and published by the Brewing Society of Japan, the Public Interest Incorporated). The raw malt extract is said to be an estimated value of a malt concentration used during the production of beer (when a sugar is added, including a sugar concentration). The raw wort extract can be measured in accordance with the SCABA (Servo Chem Automatic Beer Analyzer) method that is officially evaluated as the international laws. In the present invention, the "real extract (°P)" means a mass concentration of nonvolatile solid materials contained in the beverage, which can be measured in accordance with the BCOJ Beer Analytical Method, 8.4.3 Alcolyzer method.

The composition for foodstuff of the present invention contains D-alanine, D-isoleucine, D-leucine, and D-valine.

The mass ratio of a total content of D-isoleucine, D-leucine, and D-valine to the content of D-alanine in the composition for foodstuff of the present invention (D-isoleucine, D-leucine, and D-valine / D-alanine) is 0.1 or more, preferably 0.5 or more, more preferably 1.0 or more, even more preferably 2.0 or more, even more preferably 3.0 or more, and even more preferably 4.0 or more, from the viewpoint of making sharpness excellent, and the upper limit thereof can be 100 or less, 50 or less, and 10 or less, and the mass ratio may be within the range of any of the combinations thereof. The amount of amino acids in the composition for foodstuff as used herein is quantified as follows. IS solution and acetonitrile are added to a 5-fold dilution sample, the ingredients are blended, the mixture is then centrifuged to collect the supernatant (deproteination treatment), the sample after the deproteination treatment is subjected to a derivatization reaction under basic conditions with an amino acid derivatizing reagent (R)-BiAC, 0.1% formic acid is then added to the reaction mixture, and the mixture is subjected to LC-MS/MS analysis (Shimadzu LCMS8060).

The composition for foodstuff of the present invention can optionally contain ingredients such as carbon source raw materials (sugars), nitrogen source raw materials (peptides, amino acids), vitamins, or flavor ingredients.

The composition for foodstuff of the present invention can be used for various foodstuff such as beer-taste beverages, refreshing beverages, alcoholic beverages, green-tea beverages, and sports beverages. In particular, the composition can be suitably used in the applications of giving sharpness to beer-taste beverages. Use embodiments in these beverages will be exemplified hereinbelow.

As to the beer-taste alcoholic beverage produced using malts as raw materials, first, to a mixture containing raw materials such as wheat such as malts, optionally other grains, starches, sugars, and a bittering agent, or a colorant, and water is added an enzyme such as amylase optionally to carry out gelatinization or saccharification, and the mixture is then filtered, to provide a saccharified liquid. Hops, a bittering agent or the like is optionally added to the saccharified liquid, and a mixture is boiled, to remove solid contents such as coagulated proteins in a clearing tank. As a substitute for this saccharified liquid, hops may be added to a hot water added with a malt extract, and the mixture may be boiled. The hops may be mixed at any stage from the beginning of boiling to before the termination of boiling. As the conditions in the saccharification step, the boiling step, the solid content removal step, and the like, known conditions may be used. As the conditions in the fermentation and storage (stored liquor) steps, known conditions may be used. The fermentation liquid obtained is filtered, and a carbon dioxide gas is added to a filtrate obtained. Thereafter, a vessel is filled, and subjected to a sterilizing step, to obtain an intended beer-taste alcoholic beverage. Here, as the alcoholic ingredient, spirits derived from grains may be further added. The spirits mean liquors obtained by fermenting grains such as wheat, rice, buckwheat, or maize as raw materials with an yeast, and then further distilling the fermented liquid. As the grains which are raw materials for spirits, wheat is preferred. The composition for foodstuff of the present invention in each of the above steps may be added at any steps up to filling, and it is preferable that the composition is mixed in the boiling step, from the viewpoint of microbial quality guarantee.

As to the beer-taste alcoholic beverage produced without using malts as raw materials, a liquid sugar containing a carbon source, a nitrogen source as an amino acid-containing material other than wheat or malts, hops, a pigment, and the like are mixed together with a hot water, to provide a liquid sugar-containing solution. The liquid sugar-containing solution is boiled. When hops are used as raw materials, the hops may be mixed with the liquid sugar-containing solution during boiling, not before the beginning of boiling. As a substitute for this saccharified liquid, hops may be added to a hot water added with an extract using raw materials other than malts, and a mixture may be boiled. The hops may be mixed at any stage from the beginning of boiling to before the termination of boiling. As the conditions in the fermentation and storage (stored liquor) steps, known conditions may be used. The fermentation liquid obtained is filtered, and a carbon dioxide gas is added to a filtrate obtained. Thereafter, a vessel is filled, and subjected to a sterilizing step, to obtain an intended beer-taste alcoholic beverage. Here, as the alcoholic ingredient, spirits derived from grains may be further added. The spirits mean liquors obtained by fermenting grains such as wheat, rice, buckwheat, or maize as raw materials with an yeast, and then further distilling the fermented liquid. As the grains which are raw materials for spirits, wheat is preferred. In each of the above steps, the composition for foodstuff of the present invention may be added at any steps up to filling, and it is preferable that the composition is mixed in the boiling step, from the viewpoint of microbial quality guarantee.

The amount of the composition for foodstuff of the present invention in the production steps for the beer-taste alcoholic beverage differs depending upon the kinds of the beer-taste alcoholic beverages. In the beer-taste alcoholic beverage with a lighter taste, the composition can be added so that D-alanine is in an amount of preferably 3.0 mg/L or less, more preferably 2.0 mg/L or less, and even more preferably 1.5 mg/L or less, and that a total content of D-isoleucine, D-leucine, and D-valine is in an amount of preferably from 0.14 to 1.55 mg/L, more preferably from 0.21 to 0.90 mg/L, and even more preferably from 0.28 to 0.45 mg/L as a measure.

As to the nonalcoholic beer-taste beverage produced by using malts as raw materials, first, to a mixture containing raw materials such as wheat such as malts, optionally other grains, starches, sugars, a bittering agent, or a colorant, and water is added an enzyme such as amylase optionally to carry out gelatinization or saccharification, and the mixture is then filtered, to provide a saccharified liquid. Hops, a bittering agent or the like is optionally added to the saccharified liquid, and a mixture is boiled, to remove solid contents such as coagulated proteins in a clearing tank. As a substitute for this saccharified liquid, hops may be added to a hot water added with a malt extract, and a mixture may be boiled. The hops may be mixed at any stage from the beginning of boiling to before the termination of boiling. As the conditions in the saccharification step, the boiling step, the solid content removal step, and the like, known conditions may be used. After boiling, the wort obtained is filtered, and a carbon dioxide gas is added to a filtrate obtained. Thereafter, a vessel is filled, and subjected to a sterilizing step, to obtain an intended nonalcoholic beer-taste beverage. In each of the above steps, the composition for foodstuff of the present invention may be added at any steps up to filling, and it is preferable that the composition is mixed in the boiling step, from the viewpoint of microbial quality guarantee.

In the case of producing a nonalcoholic beer-taste beverage without using malts as raw materials, first, a liquid sugar containing a carbon source, a nitrogen source as an amino acid-containing material other than wheat or malts, hops, a pigment, and the like are mixed together with a hot water, to provide a liquid sugar-containing solution. The liquid sugar-containing solution is boiled. When hops are used as raw materials, the hops may be mixed with the liquid sugar-containing solution during boiling, not before the beginning of boiling. A carbon dioxide gas is added to the liquid sugar-containing solution after boiling. Thereafter, a vessel is filled, and subjected to a sterilizing step, to obtain an intended nonalcoholic beer-taste beverage. In each of the above steps, the composition for foodstuff of the present invention may be added at any steps up to filling, and it is preferable that the composition is mixed in the boiling step, from the viewpoint of microbial quality guarantee.

As the amount of the composition for foodstuff of the present invention in the production steps for the nonalcoholic beer-taste beverage, the composition can be added so that D-alanine in the nonalcoholic beer-taste beverage is in an amount of preferably 2.5 mg/L or less, more preferably 2.0 mg/L or less, and even more preferably 1.0 mg/L or less, and that a total content of D-isoleucine, D-leucine, and D-valine is in an amount of preferably from 0.05 to 0.90 mg/L, more preferably from 0.08 to 0.40 mg/L, and even more preferably from 0.14 to 0.25 mg/L as a measure.

The composition for foodstuff of the present invention can be preferably exemplified by lactic acid fermented extracts and processed products thereof. These embodiments will be explained hereinbelow without intending to limit the present invention to these embodiments.

The lactic acid fermented extract includes lactic acid fermented extracts in which malt extracts, rice extracts, barley extracts, cornstarch, and the like are subjected to lactic acid fermentation. From the viewpoint of fermentation ability and tastes, an embodiment of the lactic acid fermented extract in which malt extracts are subjected to lactic acid fermentation is preferred. In this embodiment, the malt extract can be prepared by subjecting malts to a series of steps of pulverization, saccharification, filtration, boiling, and filtration, or a commercially available product such as Malt Ace 20 manufactured by ORIENTAL YEAST CO., LTD. can also be used. The lactic acid bacteria used in the preparation of the lactic acid fermented extract include *Lb. fermentum, Lb. reuteri,* and the like. An embodiment of using *Lb. fermentum* is preferred, from the viewpoint of the production ability of D-aspartic acid and D-glutamic acid. As the culture conditions, it is preferable that general conditions for lactic acid bacteria culture are applied. The culture is carried out at a temperature within the range in which the lactic acid bacteria are capable of growing, specifically from 30° to 45°C. The culture is carried out at a pH of from 3.0 to 8.0, and a pH of preferably from 3.5 to 7.0. The culture time differs depending upon the kinds of the medium used and the kinds of the substrates, and the culture time is usually from 10 to 24 hours or so, preferably cultured for overnight or longer. After the culture, the culture medium obtained is subjected to a well-known means ordinarily used in the art, for example, the procedures such as membrane filtration or centrifugation to remove an insoluble solid ingredient, irrespective of the presence or absence of the sterilizing step, to give a lactic acid fermented extract.

The content of D-alanine in the lactic acid fermented extract is preferably 14.0 mg/L or less, more preferably 13.0 mg/L or less, and even more preferably 12.0 mg/L or less, from the viewpoint of making sharpness excellent, and the lower limit thereof can be, but not particularly limited thereto, for example, 0.01 mg/L or more, and the content may be the range of any combinations thereof.

The content of D-isoleucine in the lactic acid fermented extract is preferably 1.0 mg/L or more, more preferably 2.0 mg/L or more, and even more preferably 5.0 mg/L or more, from the viewpoint of making sharpness excellent, and the content is preferably 40 mg/L or less, more preferably 15 mg/L or less, and even more preferably 10 mg/L or less, from the viewpoint of inhibiting an increase in bitterness caused by D-isoleucine itself, and the content may be within the range of any combinations thereof.

The content of D-leucine in the lactic acid fermented extract is preferably 2.0 mg/L or more, more preferably 3.0 mg/L or more, and even more preferably 4.0 mg/L or more, from the viewpoint of making sharpness excellent, and the content is preferably 20 mg/L or less, more preferably 15 mg/L or less, and even more preferably 10 mg/L or less, from the viewpoint of inhibiting an increase in bitterness caused by D-leucine itself, and the content may be within the range of any combinations thereof.

The content of D-valine in the lactic acid fermented extract is preferably 1.0 mg/L or more, more preferably 2.0 mg/L or more, and even more preferably 5.0 mg/L or more, from the viewpoint of making sharpness excellent, and the content is preferably 40 mg/L or less, more preferably 15 mg/L or less, and even more preferably 10 mg/L or less, from the viewpoint of inhibiting an increase in bitterness caused by D-valine itself, and the content may be within the range of any combinations thereof.

A total content of D-isoleucine, D-leucine, and D-valine in the lactic acid fermented extract is preferably 4.0 mg/L or more, more preferably 7.0 mg/L or more, and even more preferably 14 mg/L or more, from the viewpoint of making sharpness excellent, and the total content is preferably 100 mg/L or less, more preferably 45 mg/L or less, and even more preferably 30 mg/L or less, from the viewpoint of inhibiting the generation of bitterness, and the total content may be within the range of any combinations thereof.

The processed products of the lactic acid fermented extract include concentrates, dilutions, lyophilized products, concentrated dried products, purified products subjected to fractionation or purification treatment, and the like of the lactic acid fermented extract.

### EXAMPLES

The present invention will be specifically described hereinbelow by the Examples, without intending to limit the scope of the present invention to the following Examples.

### Example 1

Malt extracts were fermented with a specified lactic acid bacterium, to give a lactic acid fermented extract. Specifically, 2.5 g of malt extracts "Malt Ace 20" manufactured by ORIENTAL YEAST CO., LTD., 0.1 g of L-aspartic acid, 0.1 g of L-glutamic acid, and 96.3 g of water were mixed, and the mixture was sterilized in a water bath at 90°C for 1 minute, and then cooled to a culture temperature for the lactic acid bacteria. To a cooled raw material mixture was inoculated with 1 g of a suspension of 1 × 10⁸ (cfu/g) commercially available lactic acid bacteria *Lb. fermentum,* and the incubation was carried out under general culture conditions for the lactic acid bacteria, to carry out lactic acid fermentation. A liquid mixture after the lactic acid fermentation was centrifuged to remove insoluble solid ingredients, to give a composition for foodstuff of Example 1 (Lactic acid fermented extract 1).

### Comparative Example 1

The same procedures as in Example 1 were carried out except that rice extracts "Hakko-mai Ekisu (fermented rice extracts)" manufactured by TAIYO CORPORATION was used in place of the malt extracts, that *Pediococcus acidilactici (Ped. acidilactici)* was used as lactic acid bacteria, and that the culture conditions were changed to 37°C for 22 hours, to give a composition for foodstuff of Comparative Example 1 (Lactic acid fermented extract 2). The details of the compositions for foodstuff obtained are shown in Table 1.

### [Table 1]

**Table 1**

| | Ex. 1 | Comp. Ex. 1 |
|---|---|---|
| (1) D-Ala, mg/L | 11.9 | 14.2 |
| (2) D-Ile, mg/L | 21.8 | 0.4 |
| (3) D-Leu, mg/L | 22.2 | 0.3 |
| (4) D-Val, mg/L | 9.5 | 0.1 |
| Total amount of (2) to (4), mg/L | 53.5 | 0.8 |
| ((2) + (3) + (4))/(1) | 4.5 | 0.06 |

A lactic acid fermented extract of each of Examples and Comparative Examples was added in an amount listed in Table 2 to 100 parts by mass of a commercially available beer-taste alcoholic beverage having a malt ratio of from 10 to 35%. The relative scoring was carried out on the bases of the following criteria by defining the evaluation of the beverage before addition of the lactic acid fermented extract as "1 point." The scoring was carried out with an increment of 0.25, and a mean score of six specialized panelists was calculated. On the basis of this mean score, the extent of the enhanced effects of the sharpness was indicated by symbols "X" to "⊚," in accordance with the following criteria. When the judgments were "△" or higher, it is found that the sharpness is enhanced. The evaluation of the beverage before addition of the lactic acid fermented extract was referred to as Reference Example 1. The results are shown in Table 2.

### (Scores for Sharpness)

1: Not tasted at all;
2: Faintly tasted;
3: Distinctively tasted; and
4: Highly Strongly tasted.

### (Evaluation Criteria for Sharpness)

×: Score for sharpness is 1.0 or more and less than 1.5;
△: Score for sharpness is 1.5 or more and less than 2.5;
○: Score for sharpness is 2.5 or more and less than 3.5; and
⊚ Score for sharpness is 3.5 or more.

### [Table 2]

**Table 2**

| | Ref. Ex. 1 (without addition) | Level 1 | Level 2 | Level 3 |
|---|---|---|---|---|
| Lactic acid fermented extract of Ex. 1, parts by mass | 0 | 0.3 | 1.0 | 0 |
| Lactic acid fermented extract of Comp. Ex. 1, parts by mass | 0 | 0 | 0 | 1.0 |
| Sharpness | × | ○ | ⊚ | × |

A lactic acid fermented extract of each of Examples and Comparative Examples was added in an amount listed in Table 3 to 100 parts by mass of a commercially available nonalcoholic beer-taste beverage. The relative scoring was carried out on the bases of the following criteria by defining the evaluation of the beverage before addition of the lactic acid fermented extract as "1 point." The scoring was carried out with an increment of 0.25, and a mean score of six specialized panelists was calculated. On the basis of this mean score, the extent of the enhanced effects of the sharpness was indicated by symbols "×" to "⊚," in accordance with the following criteria. When the judgments were " △" or higher, it is found that the sharpness is enhanced. The evaluation of the beverage before addition of the lactic acid fermented extract was referred to as Reference Example 2. The results are shown in Table 3.

### (Scores for Sharpness)

1: Not tasted at all;
2: Faintly tasted;
3: Distinctively tasted; and
4: Highly Strongly tasted.

### (Evaluation Criteria for Sharpness)

×: Score for sharpness is 1.0 or more and less than 1.5;
△: Score for sharpness is 1.5 or more and less than 2.5;
○: Score for sharpness is 2.5 or more and less than 3.5; and
⊚: Score for sharpness is 3.5 or more.

### [Table 3]

**Table 3**

| | Ref. Ex. 2 (without addition) | Level 4 | Level 5 | Level 6 |
|---|---|---|---|---|
| Lactic acid fermented extract of Ex. 1, parts by mass | 0 | 0.3 | 1.0 | 0 |
| Lactic acid fermented extract of Comp. Ex. 1, parts by mass | 0 | 0 | 0 | 1.0 |
| Sharpness | × | ○ | ⊚ | × |

It can be seen from Tables 2 and 3 that the beer-taste alcoholic beverages of Levels 1 and 2 and the nonalcoholic beer-taste beverages of Levels 4 and 5, each in which the lactic acid fermented extract of Example 1 was added, have excellent sharpness.

To each of a commercially available beer-taste alcoholic beverage having a malt ratio of from 10 to 35% (Reference Example 1) and a commercially available nonalcoholic beer-taste beverage (Reference Example 2) were added D-alanine manufactured by nacalai tesque, D-isoleucine manufactured by nacalai tesque, D-leucine manufactured by nacalai tesque, and D-valine manufactured by nacalai tesque each in an amount so as to have a content as listed in Tables 4 and 5, and the sharpness was evaluated in the same manner as in Tables 2 and 3.

### [Table 4]

**Table 4**

| | Ref. Ex. 1 | Level 7 | Level 8 | Level 9 | Level 11 | Level 12 | Level 13 | Level 14 |
|---|---|---|---|---|---|---|---|---|
| (1) D-Ala, mg/L | 0.11 | 0.11 | 0.11 | 0.11 | 0.11 | 0.60 | 1.50 | 3.00 |
| (2) D-Ile, mg/L | 0.01 | 0.07 | 0.01 | 0.01 | 0.07 | 0.07 | 0.07 | 0.07 |
| (3) D-Leu, mg/L | 0.05 | 0.05 | 0.14 | 0.05 | 0.14 | 0.14 | 0.14 | 0.14 |
| (4) D-Val, mg/L | 0.01 | 0.01 | 0.01 | 0.07 | 0.07 | 0.07 | 0.07 | 0.07 |
| Total amount of (2) to (4), mg/L | 0.07 | 0.13 | 0.16 | 0.13 | 0.28 | 0.28 | 0.28 | 0.28 |
| Sharpness | × | ⊚ | ⊚ | ⊚ | ⊚ | ⊚ | ⊚ | △ |

### [Table 5]

**Table 5**

| | Ref. Ex. 2 | Level 15 | Level 16 | Level 17 | Level 18 | Level 19 | Level 20 | Level 21 |
|---|---|---|---|---|---|---|---|---|
| (1) D-Ala, mg/L | 0.05 | 0.05 | 0.05 | 0.05 | 0.05 | 0.30 | 1.00 | 3.00 |
| (2) D-Ile, mg/L | 0.00 | 0.03 | 0.00 | 0.00 | 0.03 | 0.03 | 0.03 | 0.03 |
| (3) D-Leu, mg/L | 0.01 | 0.01 | 0.05 | 0.01 | 0.05 | 0.05 | 0.05 | 0.05 |
| (4) D-Val, mg/L | 0.00 | 0.00 | 0.00 | 0.05 | 0.05 | 0.05 | 0.05 | 0.05 |
| Total amount of (2) to (4), mg/L | 0.01 | 0.04 | 0.05 | 0.06 | 0.14 | 0.14 | 0.14 | 0.14 |
| Sharpness | × | ⊚ | ⊚ | ⊚ | ⊚ | ⊚ | ○ | × |

It can be seen from Tables 4 and 5 that the sharpness is enhanced when D-isoleucine, D-leucine, and D-valine are added, as compared to that of without addition. In addition, it can be seen that the sharpness tends to be worsened as the content of D-alanine becomes larger.

### INDUSTRIAL APPLICABILITY

The composition of the present invention is useful in the fields of foodstuff, and the like.

## Claims

1. A composition for foodstuff, having a mass ratio of a total content of D-isoleucine, D-leucine, and D-valine to the content of D-alanine (D-isoleucine, D-leucine, and D-valine / D-alanine) of 0.1 or more.

2. The composition for foodstuff according to claim 1, wherein the content of D-alanine is 14.0 mg/L or less, and wherein the content of D-isoleucine is 1.0 mg/L or more.

3. The composition for foodstuff according to claim 1 or 2, wherein the content of D-alanine is 14.0 mg/L or less, and wherein the content of D-leucine is 1.0 mg/L or more.

4. The composition for foodstuff according to any one of claims 1 to 3, wherein the content of D-alanine is 14.0 mg/L or less, and wherein the content of D-valine is 1.0 mg/L or more.

5. The composition for foodstuff according to any one of claims 1 to 4, wherein the content of D-alanine is 14.0 mg/L or less, and wherein a total content of D-isoleucine, D-leucine, and D-valine is 1.0 mg/L or more.

6. The composition for foodstuff according to any one of claims 1 to 5, which is a lactic acid fermented extract or a processed product thereof.

7. The composition for foodstuff according to claim 6, wherein the lactic acid fermented extract is obtained by a method comprising fermenting a malt extract with *Lb. fermentum.*

8. The composition for foodstuff according to any one of claims 1 to 7, for use in beer-taste beverages.

9. A method for producing a composition for foodstuff as defined in any one of claims 1 to 8, comprising fermenting a malt extract with *Lb. fermentum.*
